# EUROPEAN PATENT APPLICATION

(11) **EP 4 529 880 A2**
(43) Date of publication of application: **02.04.2025**
(21) Application number: 24203103.7
(22) Date of filing: 27.09.2024
(51) Int. Cl.: A61B 34/30, A61B 34/37, A61B 34/00, A61M 25/01, A61B 17/00

(54) **ENDOLUMINAL ROBOTIC CATHETER CONTROL SYSTEM**

(30) Priority: 29.09.2023 US 202363541383 P; 12.09.2024 US 202463694134 P
(71) Applicant: Covidien LP, Mansfield, MA 02048 (US)
(72) Inventor: BLANCO, Matthew C., Boston, 02210 (US); THOMSON, Jonathan Scott, Boston, 02210 (US); CENICCOLA, Anthony L., North Haven, 06473 (US); ZINSER, Benjamin T., Boston, 02210 (US)
(74) Representative: Maschio & Soames IP Ltd

(57) **Abstract**

A catheter drive system including a motor pack having a plurality of motors, each motor connected to a driver, a catheter quick connect, having a catheter mounted therein, and rotary interfaces configured to mate with the drivers, a plurality of spindles, each spindle coupled to one of the rotary interfaces such that rotation of the rotary interface is transferred to the spindle, a plurality of pull-wires, each pull-wire connected to one of the spindles and extending into a distal portion of the catheter, a Z-drive quick connect configured to receive the catheter and move the catheter along its longitudinal axis, and a Z-drive including at least one motor and a plurality of drivers mechanically coupled to the motor, each driver coupleable to a rotary interface on the Z-drive quick connect, wherein rotation of the drivers by the motor is transferred to the rotary interfaces of the Z-drive quick connect.

## Description

### BACKGROUND

### Technical Field

This disclosure relates to the field of medical device navigation, and in particular, to control systems for navigating a medical device to an area of interest.

### Description of Related Art

There are several commonly applied medical methods, such as endoscopic procedures or minimally invasive procedures, for treating various maladies affecting organs including the liver, brain, heart, lungs, gall bladder, kidneys, and bones. Often, one or more imaging modalities, such as magnetic resonance imaging (MRI), ultrasound imaging, computed tomography (CT), or fluoroscopy are employed by clinicians to identify and navigate to areas of interest within a patient and ultimately a target for biopsy or treatment. In some procedures, pre-operative scans may be utilized for target identification and intraoperative guidance. However, real-time imaging may be required to obtain a more accurate and current image of the target area. Furthermore, real-time image data displaying the current location of a medical device with respect to the target and its surroundings may needed to navigate the medical device to the target in a save and accurate manner (*e.g.*, without causing damage to other organs or tissue).

For example, an endoscopic approach has proven useful in navigating to areas of interest within a patient, and particularly so for areas within luminal networks of the body such as the lungs, blood vessels, colorectal cavities, and the renal ducts. To enable the endoscopic approach, navigation systems have been developed that use previously acquired MRI data or CT image data to generate a three-dimensional (3D) rendering, model, or volume of the particular body part.

The resulting volume generated from the MRI scan or CT scan may be utilized to create a navigation plan to facilitate the advancement of a navigation catheter (or other suitable medical device) through a bronchoscope and a branch of the bronchus of a patient to an area of interest. A locating or tracking system, such as an electromagnetic (EM) tracking system, may be utilized in conjunction with, for example, CT data, to facilitate guidance of the navigation catheter through the branch of the bronchus to the area of interest. In certain instances, the navigation catheter may be positioned within one of the airways of the branched luminal networks adjacent to, or within, the area of interest to provide access for one or more medical instruments.

As will be appreciated, accurate placement of the catheter and therewith the medical instrument is important to ensure successful therapy. Improvements to the current navigation catheter systems are desired.

### SUMMARY

One aspect of the disclosure is directed to a catheter drive system including a motor pack having a plurality of motors, each motor connected to a driver; a catheter quick connect, including a catheter mounted therein, and a plurality of rotary interfaces configured to mate with the drivers; a plurality of spindles, each spindle coupled to one of the rotary interfaces such that rotation of the rotary interface is transferred to the spindle; a plurality of pull-wires, each pull-wire connected to one of the spindles and extending into a distal portion of the catheter, where rotation of the spindle in a first rotational direction retracts the pull-wire onto the spindle articulating the catheter in a first direction, and rotation of the spindle in a second rotational direction relaxes the catheter allowing another pull-wire to articulate the catheter in second direction opposite the first direction. a z-drive quick connect configured to receive the catheter and move the catheter along its longitudinal axis; a z-drive including at least one motor and a plurality of drivers mechanically coupled to the motor, each driver coupleable to a corresponding rotary interface on the z-drive quick connect, where rotation of the drivers by the motor is transferred to the rotary interfaces of the z-drive quick connect. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The catheter drive system where the catheter quick connect and the motor pack each include a plurality of attachment devices configured to affix the catheter quick connect to the motor pack. The motor pack includes an alignment device configured to align the motor pack and the catheter quick connect. The alignment device is a post configured to mate with an opening on the catheter quick connect. The alignment device is a post configured to mate with an opening on the z-drive. The z-drive quick connect and the z-drive each include a plurality of attachment devices configured to affix the catheter quick connect to the motor pack. The z-drive quick connect includes an alignment device configured to align the motor pack, and the catheter quick connect. The z-drive directly connects to a drive gear connected to a first of the plurality of drivers, and a remainder of the plurality of drivers are operably connected to the drive gear by driven gears. The z-drive quick connect includes a plurality of drive wheels, where the drive wheels are configured to frictionally drive the catheter when the drive wheels rotate. The catheter quick connect is a four pull-wire device. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. The catheter drive system including a slack management guide. The slack management guide includes a pair of angled guide plates and a profiled base plate. The profiled base plate includes a distal end configured to prevent kinking of the catheter. The angled guide plates are configured to secure a portion of the catheter therebetween to prevent twisting of the catheter.

A further aspect of the disclosure is directed to A robotic catheter drive system including a z-drive mounted at a distal end of a rail and configured to receive a z-drive quick connect; a motor adapter mounted on a proximal end of the rail, the motor adapter configured to mate with an instrument drive unit (IDU) and transmit motorized motions from the IDU to a robotic catheter quick connect and the z-drive; a slack management guide mounted on the rail between the motor adapter and the z-drive and configured to manage slack in a catheter.

Implementations may include one or more of the following features. The robotic catheter drive system may include a disposable catheter system, the disposable catheter system including a z-drive quick connect connected to a distal portion of a catheter and the z-drive and a robotic catheter quick connect on a proximal portion of the catheter and the motor adapter. The profiled base plate includes a distal end configured to prevent kinking of the catheter. The pair of angled guide plates are configured to secure at least a portion of the catheter between them to prevent twisting of the catheter. The slack management guide includes a pair of angled guide plates and a profiled base plate. The robotic catheter drive system may include a shaft mechanically coupling the adapter and the z-drive.

### BRIEF DESCRIPTION OF THE DRAWINGS

Various aspects and embodiments of the disclosure are described hereinbelow with references to the drawings, wherein:
FIG. 1 is a schematic view of a navigation system in accordance with the disclosure;
FIG. 2. depicts a quick connect control system for navigation of a catheter within a patient in accordance with the disclosure;
FIG. 3A depicts a spindle for a pull wire of a catheter quick connect of FIG. 2 in accordance with the disclosure;
FIG. 3B depicts four spindles of Fig 3A in accordance with the disclosure;
FIG. 3C depicts a spindle of FIG. 3A with a slot for receiving a pull-wire in accordance with the disclosure;
Fig, 3D depicts a spindle of FIG. 3A wound with a pull wire to be inserted into the catheter quick connect of FIG. 2 in accordance with the disclosure;
FIG. 4A depicts a housing for the catheter quick connect of FIG. 2;
FIG. 4B depicts the housing of FIG. 4A with a catheter and pull wires in accordance with the disclosure;
FIG. 4C depicts a rear view of the housing of FIG. 4A in accordance with the disclosure;
FIG. 4D depicts a top view of the housing of FIG. 4A with the spindles of FIG. 3A installed;
FIG. 4E depicts the housing of FIG. 4A with the spindles of FIG. 3A in place, an internal cover in accordance with the disclosure.
FIG. 4F depicts the housing of FIG. 4A with the spindles of FIG. 3A held in place by an internal cover in accordance with the disclosure;
FIG. 4G depicts the housing of FIG. 4A with a spindle drivers connected to each spindle of FIG. 3A in accordance with the disclosure;
FIG. 4H depicts a view of the housing of FIG. 4A and a spindle driver to be inserted into one of the spindles in accordance with the disclosure;
FIG. 4I depicts the housing of FIG. 4A with a top cover installed in accordance with the disclosure;
FIG. 4J depicts a rear perspective view of the housing of FIG. 4A in accordance with the disclosure;
FIG. 4K depicts a backside of a cover of the housing of FIG. 4A in accordance with the disclosure;
FIG. 5A depicts a fully assembled motor pack of FIG. 2 configured to receive the catheter quick connect of FIG. 2 in accordance with the disclosure;
FIG. 5B depicts an internal top view of a motor pack of FIG. 2 in accordance with the disclosure;
FIG. 5C depicts a partially assembled motor pack of FIG. 2 in accordance with the disclosure;
FIG. 6A is a top view of a Z-drive quick connect of FIG. 2 in accordance with the disclosure;
FIG. 6B is a bottom view of the Z-drive quick connect of FIG. 6A in accordance with the disclosure;
FIG. 6C depicts a comparative view of the bottom view of the Z-drive quick connect of FIG. 6A and an alternative construction in accordance with the disclosure;
FIG. 6D depicts a partially disassembled Z-drive quick connect in accordance with the disclosure;
FIG. 6E depicts a top view of an alternative Z-drive quick connect in accordance with the disclosure;
FIG. 6F depicts a bottom view of the Z-drive quick connect of FIG. 6E;
FIG. 7A depicts a top perspective view of a Z-drive in accordance with the disclosure;
FIG. 7B depicts a top perspective view of an alternative Z-drive in accordance with the disclosure;
FIG. 7C depicts the Z-drive quick connect of FIG. 6A connected to the Z-drive of FIG. 7B;
FIG. 7D depicts an alternative Z-drive in accordance with the disclosure;
FIG. 8 depicts a perspective view of the quick connect control system for navigation of a catheter within a patient of FIG. 2; and
FIG. 9 is a schematic view of a computing system in accordance with the disclosure.
FIG. 10 depicts a partial perspective view of a slack management guide in accordance with the disclosure;
FIG. 11 depicts the slack management guide of FIG. 10 connected to a motor pack in accordance with the disclosure;
FIG. 12 depicts the slack management guide of FIG. 10 connected to a motor adapter in accordance with the disclosure;
FIG. 13 depicts a catheter drive system navigating a lung model in accordance with the disclosure;
FIG. 14 depicts a dual catheter drive system assembly in accordance with the disclosure
FIG. 15 depicts an alternative view of the dual catheter drive system assembly in accordance with the disclosure;
FIG. 16 depicts a surgical robotic arm in accordance with aspects of the disclosure;
FIG. 17 depicts a catheter drive system for mounting on the surgical robotic arm of FIG. 16 in accordance with the disclosure; and
FIG. 18 depicts the catheter drive system of FIG. 17 mounted on the surgical robotic arm of FIG. 16 in accordance with the disclosure;

### DETAILED DESCRIPTION

The disclosure is directed to systems and methods of navigating a catheter within luminal networks of a patient. In particular the disclosure is directed to systems and methods for motorized or robotic catheter control for manipulation of catheters and tools within the lungs of a patient. The systems and methods of the disclosure provide a disposable catheter that can be quickly attached to a driver that enables four wire articulation and a separate driver that enables driving of the catheter into the patient (i.e., the Z-direction). As a result of the quick connect system described herein, catheters of different sizes can be employed from procedure to procedure, or even within the same procedure without requiring any alteration of the drive mechanisms. These motorized or robotic navigation systems and methods can be coupled to existing navigation systems including electromagnetic (EM) navigation systems such as the ILLUMISITE^{®} system currently offered by Medtronic.

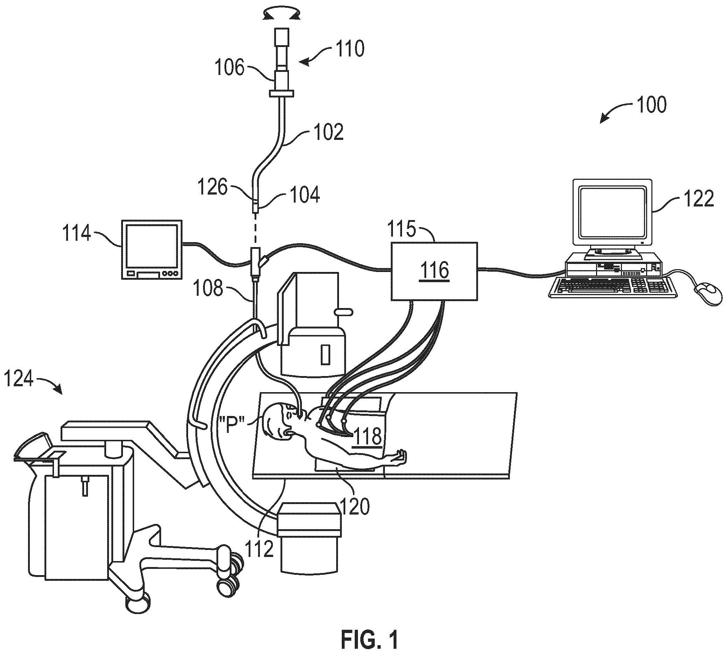

With reference to FIG. 1, a navigation system facilitating navigation of a medical device through a luminal network and to an area of interest is illustrated and generally identified by reference numeral 100. The navigation system 100 includes a catheter 102 and a guide assembly 106. In one embodiment, the catheter 102 is inserted into a bronchoscope 108 for access to a luminal network of the patient P. In this manner, the catheter 102 may be inserted into a working channel of the bronchoscope 108 for navigation through a patient's luminal network, such as the lungs. The catheter 102 may itself include imaging capabilities via an integrated camera or optics component 109 and therefore, a separate bronchoscope 108 is not strictly required. A locatable guide (LG) 110 (a second catheter), including a sensor 104 may be inserted into the catheter 102 and locked into position such that the sensor 104 extends a desired distance beyond a distal tip of the catheter 102. As can be appreciated, the position and orientation of the sensor 104 relative to a reference coordinate system, and thus the distal portion of the catheter 102, within an electromagnetic field can be derived. Catheter guide assemblies 106 are currently marketed and sold by Medtronic PLC under the brand names SUPERDIMENSION^{®} Procedure Kits, or EDGE^{®} Procedure Kits, and are contemplated as being usable with the disclosure.

Continuing with FIG. 1, the system 100 generally includes an operating table 112 configured to support a patient P and monitoring equipment 114 coupled to the bronchoscope 108 or catheter 102 (*e.g*., a video display, for displaying the video images received form the video imaging system of the bronchoscope 108 or the catheter 102), a locating or tracking system 115 including a tracking module 116, a plurality of reference sensors 118 and a transmitter mat 120 including a plurality of incorporated markers, and a computing device 122 including software and/or hardware used to facilitate identification of a target, pathway planning to the target, navigation of a medical device to the target, and/or confirmation and/or determination of placement of the catheter 102, or other suitable device therethrough, relative to the target.

A six degrees-of-freedom electromagnetic locating or tracking system 115, or other suitable system for determining position and orientation of a distal portion of the catheter 102, is utilized with the LG 110, as will be described in further detail hereinbelow, for performing registration of a detected position of the sensor 104 and a 3D model generated from a CT or MRI image scan. The tracking system 115 is configured for use with the LG 110 and particularly with the sensor 104. As described hereinabove, the LG 110 and the sensor 104 are configured for insertion through the catheter 102 into the patient P's airways (either with or without the bronchoscope 108) and are selectively lockable relative to one another via a locking mechanism (not shown).

With continued reference to FIG. 1, the transmitter matt 120 is positioned beneath the patient P. The transmitter matt 120 generates an electromagnetic field around at least a portion of the patient P within which the position of the plurality of reference sensors 118 and the sensor 104 can be determined with the use of the tracking module 116. A second electromagnetic sensor 126 may also be incorporated into the end of the catheter 102. The second electromagnetic sensor 126 may be a five degree-of-freedom sensor or a six degree-of-freedom sensor. One or more of the reference sensors 118 are attached to the chest of the patient P. Registration is generally performed to coordinate locations of the 3D model and 2D images from the planning phase, with the patient P's airways as observed through the bronchoscope 108 and allow for the navigation phase to be undertaken with knowledge of the location of the sensor 104.

Registration of the patient P's location on the transmitter mat 120 may be performed by moving the sensor 104 through the airways of the patient P. In this manner, data pertaining to locations of the sensor 104, while the LG 110 is moving through the airways, is recorded using the transmitter mat 120, the references sensors 118, and the tracking system 115. A shape resulting from this location data is compared to an interior geometry of passages of a 3D model, and a location correlation between the shape and the 3D model based on the comparison is determined, *e.g.,* utilizing the software on the computing device 122. In addition, the software identifies non-tissue space (*e.g.,* air filled cavities) in the 3D model. The software aligns, or registers, an image representing a location of the sensor 104 with the 3D model and/or 2D images generate from the 3D model, which are based on the recorded location data and an assumption that the LG 110 remains located in non-tissue space in patient P's airways. Alternatively, a manual registration technique may be employed by navigating the bronchoscope 108 with the sensor 104 to prespecified locations in the lungs of the patient P, and manually correlating the images from the bronchoscope 108 to the model data of the 3D model.

Although generally described with respect to EMN systems using EM systems, the instant disclosure is not so limited and may be used in conjunction with flexible sensors, such as fiber-bragg grating sensors, inertial measurement units (IMU), ultrasonic sensors, without sensors, or combinations thereof. As outlined below, it is contemplated that the devices and systems described herein may be used in conjunction with robotic systems such that robotic actuators drive the catheter 102 or bronchoscope 108 proximate the target.

In accordance with aspects of the disclosure, the visualization of intra-body navigation of a medical device (*e.g.,* a biopsy tool or a therapy tool), towards a target (*e.g.,* a lesion) may be a portion of a larger workflow of a navigation system. An imaging device 124 (*e.g.,* a CT imaging device such as a cone-beam computed tomography (CBCT) device, including but not limited to Medtronic plc's O-arm^{™} system) capable of acquiring 2D and 3D images or video of the patient P is also included in the particular aspect of system 100. The images, sequence of images, or video captured by imaging device 124 may be stored within the imaging device 124 or transmitted to the computing device 122 for storage, processing, and display. In embodiments, the imaging device 124 may move relative to the patient P so that images may be acquired from different angles or perspectives relative to the patient P to create a sequence of images, such as a fluoroscopic video. The pose of the imaging device 124 relative to the patient P while capturing the images may be estimated via markers incorporated with the transmitter mat 120. The markers are positioned under the patient P, between the patient P and the operating table 112, and between the patient P and a radiation source or a sensing unit of the imaging device 124. The markers incorporated with the transmitter mat 120 may be two separate elements which may be coupled in a fixed manner or alternatively may be manufactured as a single unit. It is contemplated that the imaging device 124 may include a single imaging device or more than one imaging device.

Continuing with FIG. 1, the computing device 122 may be any suitable computing device including a processor and a storage medium, wherein the processor is capable of executing instructions stored on the storage medium. The computing device 122 may further include a database configured to store patient data, CT data sets including CT images, fluoroscopic data sets including images and video, 3D reconstructions, navigation plans, and any other such data. Although not explicitly illustrated, the computing device 122 may include inputs, or may otherwise be configured to receive CT data sets, fluoroscopic images/video, and other data described herein. The computing device 122 includes a display configured to display graphical user interfaces. In embodiments, the computing device 122 may be connected to one or more networks through which one or more databases may be stored.

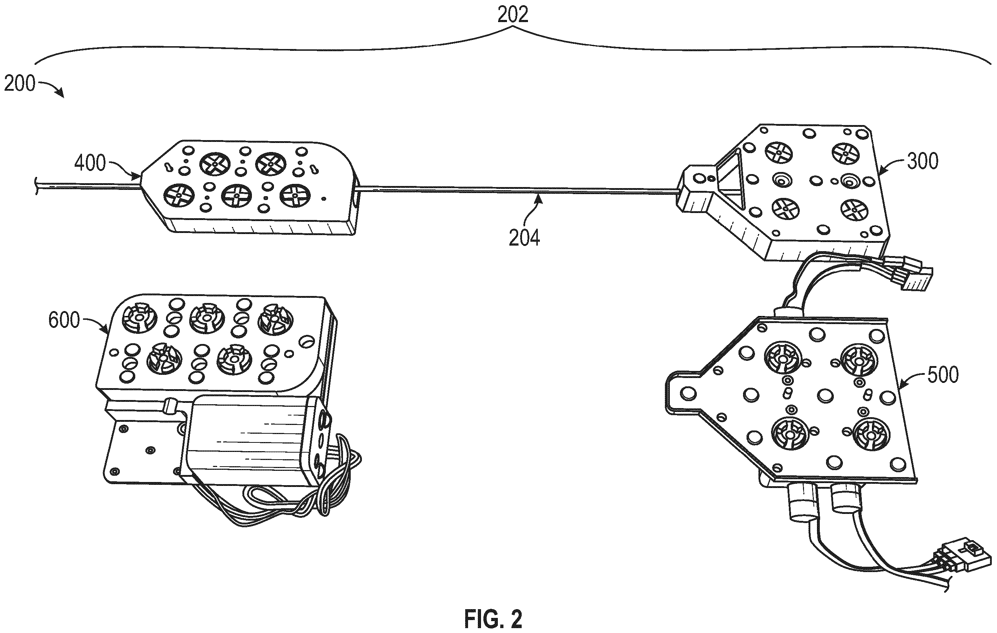

FIG. 2 depicts a catheter drive system 200 in accordance with the disclosure. The catheter drive system 200 includes a disposable catheter system 202 that is comprised of a catheter 204, a catheter quick connect 300 and a Z-drive quick connect 400. The disposable catheter system 202 is configured to be received on two separate drive mechanisms. The first drive mechanism is a motor pack 500, which manipulates the catheter quick connect 300 to enable articulation of a distal portion of the catheter 202 (e.g., up, down, left, right). The second drive mechanism is the Z-drive 600, which is configured to mate with the Z-drive quick connect 400 to drive the catheter 202 into the patient (i.e., the Z-direction). Each of these sub-components is described in greater detail below.

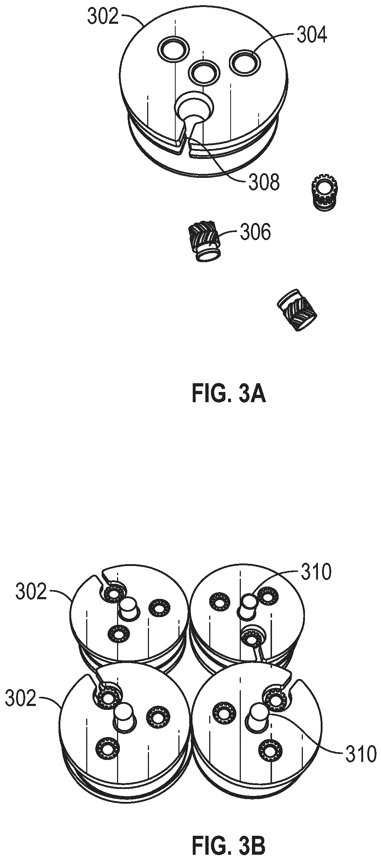

FIG. 3A depicts a spindle 302. The spindle 302 includes three openings 304 for receiving a thread insert 306. One of the openings 304 includes a slot 308 configured to receive a pull wire (described below). FIG. 3B depicts four spindles 302 with the thread inserts 306 inserted into each of the openings 304 of each spindle 302. In addition, a post 310 is inserted into an opening formed in the spindle 302 to provide an axis of rotation for the spindle 302 as described in greater detail below.

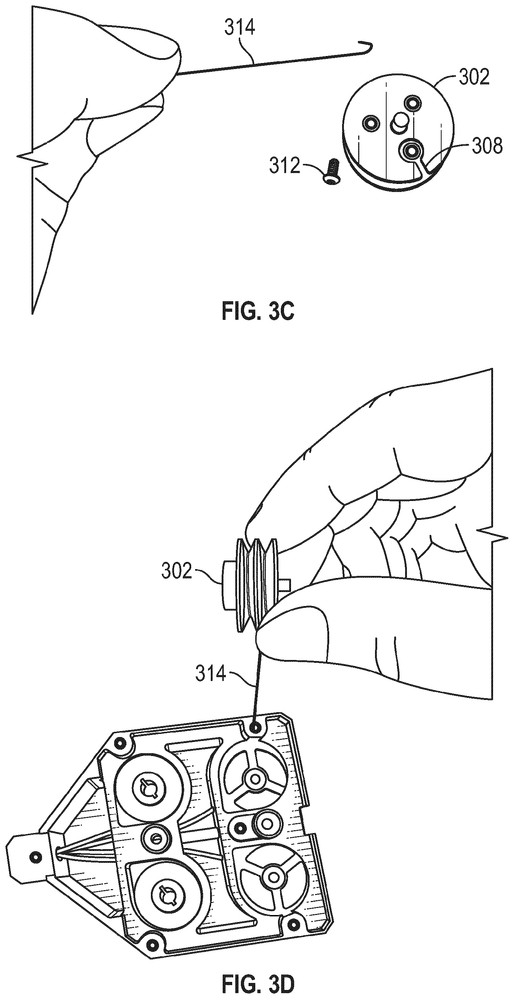

FIG. 3C depicts a spindle 302, a set screw 312 and a pull wire 314. In practice a hook is formed in the end of the pull wire 314 and the pull wire 314 is held in the opening 304 with the slot 308 by the set screw 312. The pull wires 314 can then be wound around the spindle 302, as shown in FIG. 3D. The opposite end of the pull wire 314 may be secured to a pull ring or other feature in the distal portion of the catheter 204 such that changes in length of the pull wire 314 causes changes in the shape of the catheter 204.

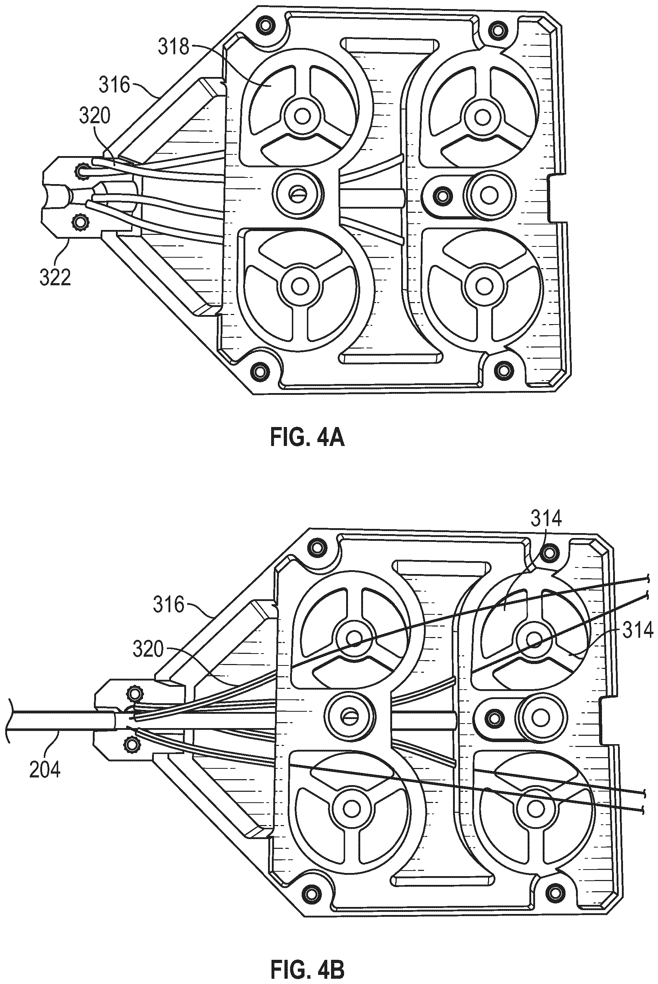

FIG. 4A depicts a housing 316 of the catheter quick connect 300. The housing 316 includes four openings 318 each configured to receive a spindle 302. Four wire guides 320 are provided. One wire guide 318 extends from each of the openings 318 to a catheter clamp 322. The wire guides 320 are configured to allow the pull wires 314 to extend from a proximal portion of the catheter 204 to the opening 318 to prevent the pull wires 314 from entangling each other and other elements of the catheter quick connect 300. FIG. 4B depicts the housing 416 with the catheter 204 inserted and the pull wires 314 extending through each of the wire guides 320.

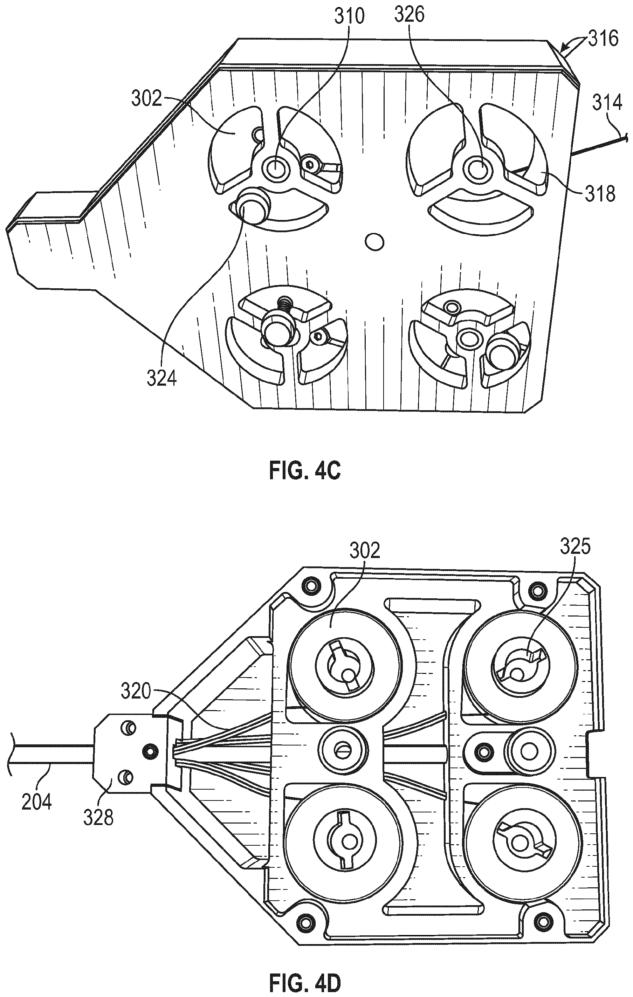

As each spindle 302 has a pull wire 314 wound thereon, the spindle can be inserted into the housing 316 as depicted in FIG. 4C. FIG. 4C depicts a bottom view of the catheter quick connect 300. Thumb screws 324 may be threaded into one of the thread inserts 306 and prevent the spindle from rotating about its shaft 310, which has been inserted into a bushing 326 in the bottom of the opening 318 into which the spindle 302 has been inserted.

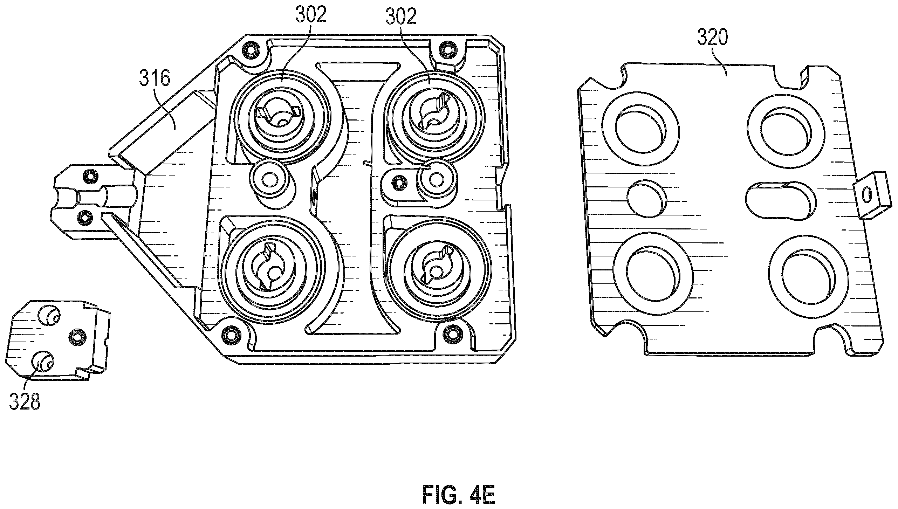

FIG. 4D depicts a top view of the housing 416 after insertion of the spindles 302 into the openings 318 after winding of the pull wires 314 thereon. Each spindle 302 includes an opening 325 (e.g., a slotted opening) formed therein. The catheter 204 is held in place on the housing by clamping member 326 which mates with the catheter clamp 322 to secure the catheter 204 therebetween. FIG. 4E depicts the housing 416 with the spindles 302 inserted into the openings 318, but with the catheter 204 and the wire guides 320 removed. Fig, 4E also shows an internal cover 330 which is configured to hold all four of the spindles in the housing 316. FIG. 4E also provides a view of the clamping member 328.

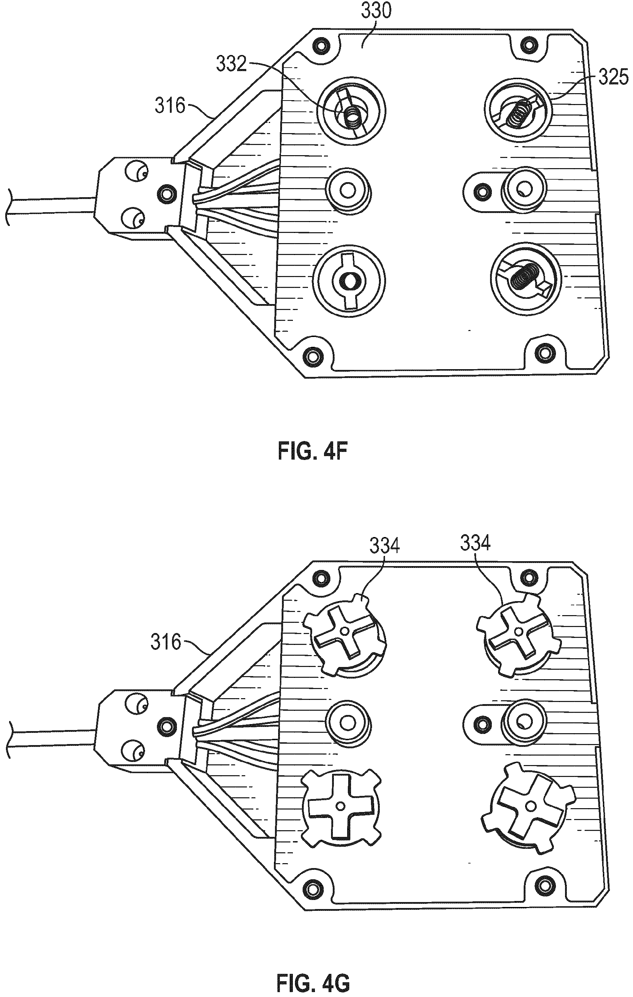

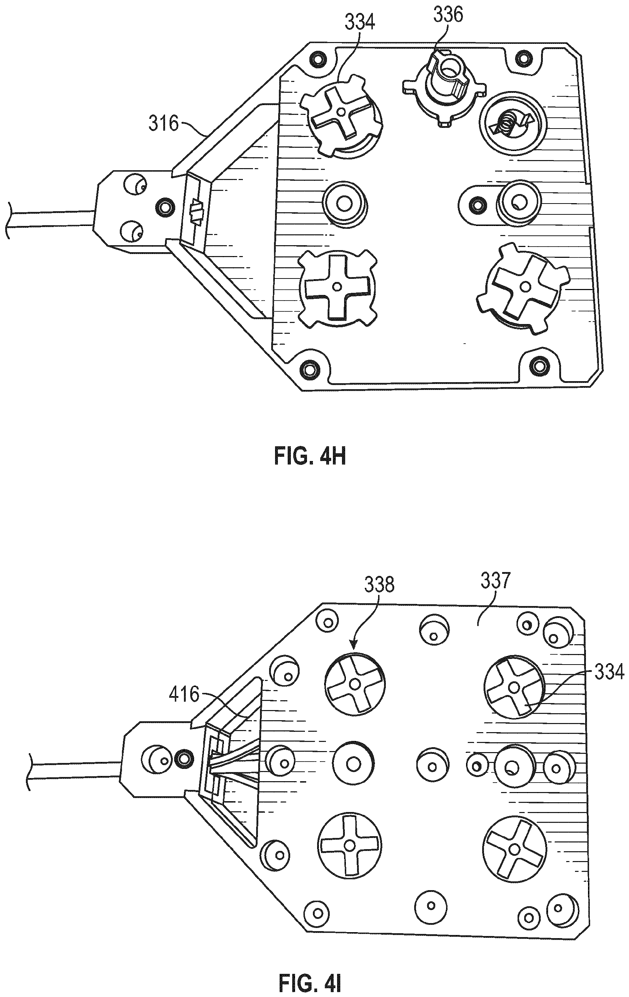

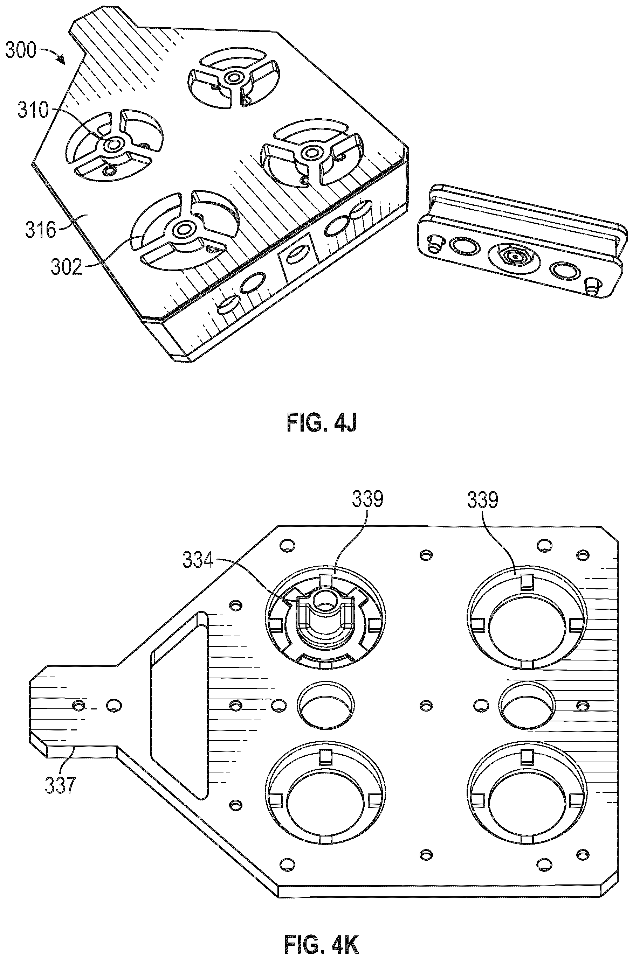

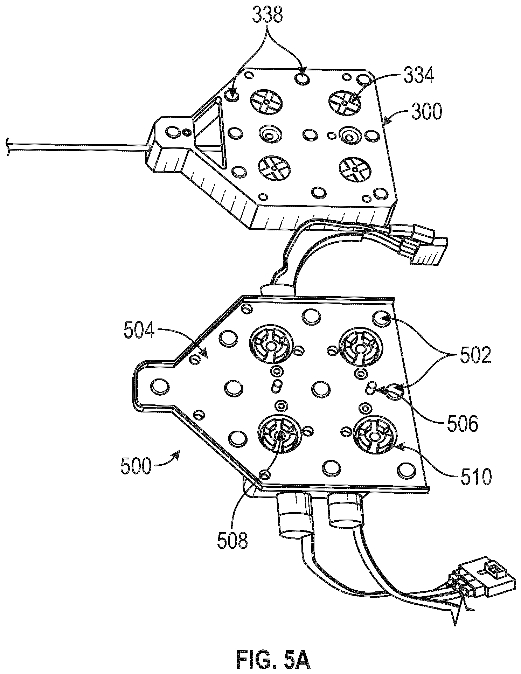

FIG. 4F depicts a top view of the housing 316 with the internal cover 330 placed over the spindles such that the spindles 302, and particularly the openings 325 in the spindles 302 are accessible. Springs 332 are placed in each of the openings 325 of the spindles 302. FIG. 4G depicts the housing 316 with a rotary interface 334 inserted into each of the openings 325. The rotary interfaces 334 are depicted here as having a cross or plus-sign shape, however other shapes are considered within the scope of the disclosure. FIG. 4H depicts one aspect of the rotary interface 334, where a portion of the rotary interface has protrusions 336 that mate with the openings 325. As shown, the protrusions 336 fit within the slot in the opening 325, and the protrusions 336 transfer any rotation motion imparted on the rotary interfaces 334 (described below) to the spindles 302. In this manner, rotation of the rotary interfaces 334 causes the pull wires 314 to be taken up onto the spindle 302 or paid out from the spindle 302, depending on the direction of rotation. As will be appreciated, in a four pull-wire system, the pull-wires 314 operate in pairs where when the catheter 204 is to be articulated left, the pull-wire 314 for left articulation is taken up onto the spindle 302, and at the same time the spindle 302 for right articulation pays its pull-wire 314 out. FIG. 4I depicts a cover 337 placed onto the housing 316. The rotary interfaces 334 remain accessible through openings 338 in the cover 336. The springs 332 bias the rotary interfaces 334 towards the cover 336 to limit movement of the rotary interfaces 334 within the housing 316 and cover 337. FIG. 4J depicts the final catheter quick connect 300 from a bottom perspective view with the shafts 310 extending through the bushings 326 and the thumb screws 312 removed. In this configuration, the catheter quick connect 300 is ready for use. FIG. 4K depicts an underside of the cover 337. When assembled, the springs 325 act on the rotary interface 337 and bias the rotary interface against the underside of the cover 337. Protrusions 339 on the underside of the cover 337 interact with the rotary interfaces 334 and prevent the unspooling of the pull wires 314. When the catheter quick connect 300 is not mounted on the motor pack 500. Upon placement of the catheter quick power 300 on the motor pack 500 the drivers 508 (FIG. 5A) act on the rotary interfaces 337 and compress the spring 325 such that the rotary interface 337 clears the protrusions 339 allowing the rotary interfaces 337 to rotate freely as driven by the drivers 508.

FIG. 5A depicts the catheter quick connect 300, described above ready to be mounted on a motor pack 500. The catheter quick connect 300 includes a number of securing elements 338, for example magnets, which mate with corresponding securing elements 502 mounted in a top plate 504 of the motor pack 500. The top plate 504 of the motor pack 500 includes one or more alignment features 506 (e.g., pins) which are configured to be received in openings 340 in the cover 336 of the quick catheter connect 300. The alignment features 402 and openings 340 ensure that the motor pack 500 and the quick catheter connect 300 are properly aligned while the securing elements 338 and 502 hold the two firmly together and prevent their relative movement during use.

Openings 510 in the top plate 504 allow drivers 508 to extend through the top plate 504. The drivers 508 are configured to mate with the rotary interfaces 334 of the catheter quick connect 300. The drivers 508 are each individually driven by a motor and gear mechanism, described below. Operation of one or more of these drivers 508 produces rotation of the corresponding spindle 302 in the catheter quick connect 300 vis the rotary interfaces 334 described above.

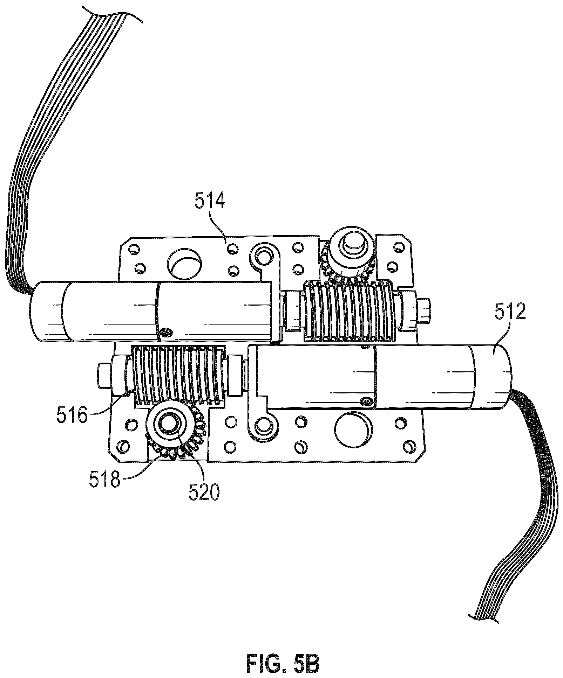

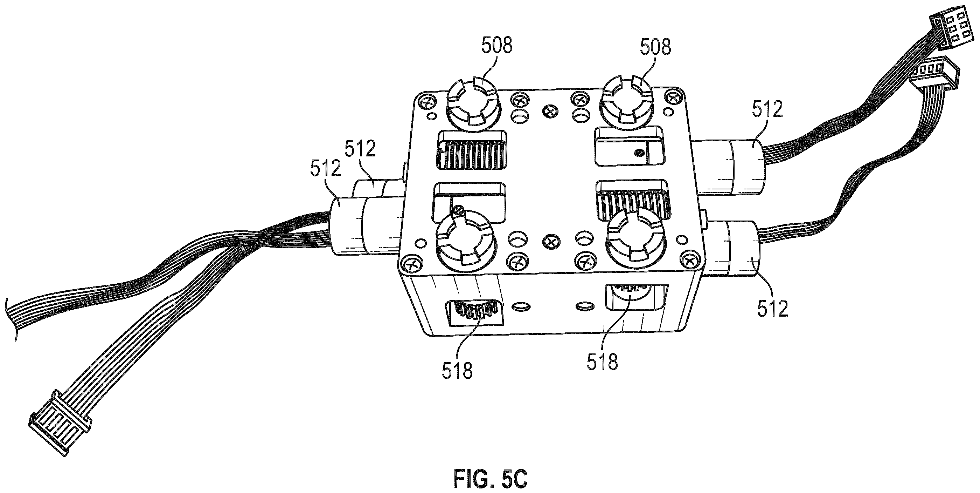

FIG. 5B depicts one half of a drive mechanism of the motor pack 500. Two motors 512 are mounted on a base 514. The motors 512 drive a screw gear 516 that mates with a second gear 518, with a shaft 520 extending from the second gear 518. A second half of the drive mechanism of the motor pack 500 is substantially similar, but with an opposite orientation of the motors 512, as can be seen in FIG. 5C. The two halves mount together and four drivers 508 are mounted to each of the four shafts 520 geared to the four screw gears 516. Securing the top plate to the assembly depicted in FIG. 5C completes the motor pack 500, which is then configured to receive the catheter quick connect 300.

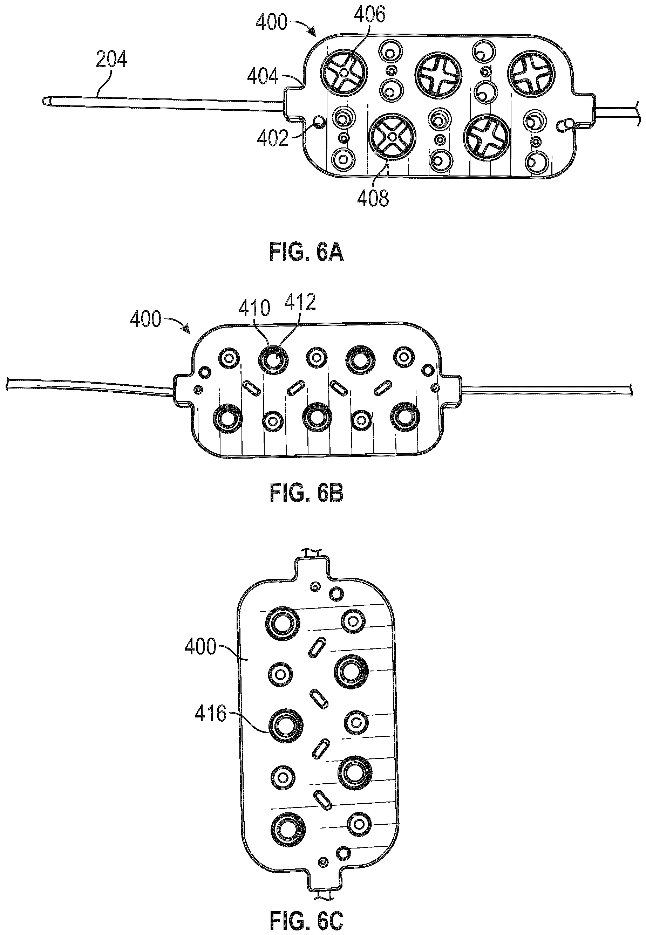

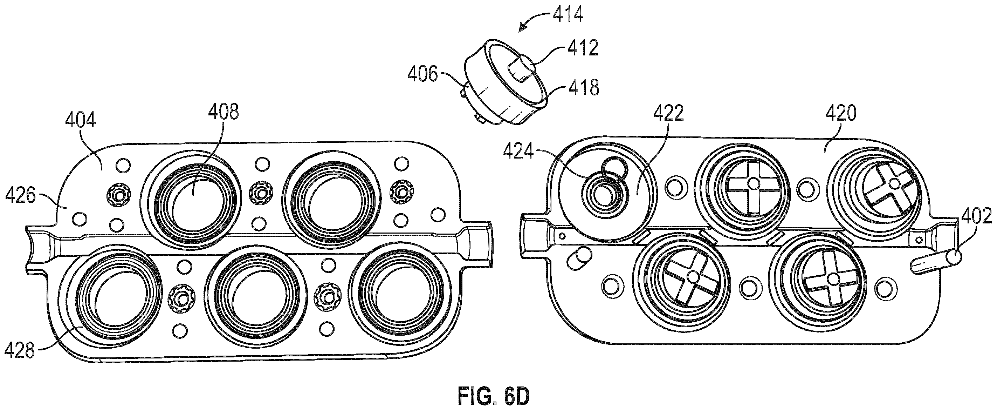

FIG. 6A depicts a top view of a Z-drive quick connect 400 in accordance with the disclosure. The catheter 204 extends through the Z-drive quick connect 400. Alignment pins 402 extend from a top plate 404 of the Z-drive quick connect 400 and mate with openings on the Z-drive 600 described herein below. Rotary interfaces 406, which are similar to the rotary interfaces 334 extend through openings 408 in the top plate 404. Again, the rotary interfaces 406 have a cross or plus shape but are not so limited and may employ other designs. FIG. 6B depicts a bottom view of the Z-drive quick connect 400. In the embodiment of FIG. 6B, bearings 410 (e.g., ball or roller bearings) are configured to receive a shaft 412 of a drive wheel 414 (See FIG. 6D). FIG. 6C depicts an alternative arrangement where the shafts 412 of the drive wheels 414 are received in a sliding bearing 416 formed of a lubricious material such as PTFE, Delrin^{®}, etc.

FIG. 6D depicts the Z-drive quick connect 400 in a partially disassembled view. As can be seen a top plate 404 includes a plurality of openings 408. Each opening 408 is sized to allow access of the rotary interfaces 406 of the drive wheel 414. A channel 416 between the openings 408 is sized and placed to allow for passage of the catheter 204, but also force the catheter into frictional contact with a friction surface 418 of the drive wheels 414. As shown the drive when 414 and the friction surface 418 may have a concave shape to ensure the catheter 204 can pass the multiple offset drive wheels 414. A bottom plate 420, shown with 4 of 5 of the drive wheels 414 received in recesses 422 formed therein. At the bottom of each recess 422 is a bearing 424 which is configured to receive the shaft 412 about which the drive wheel 414 rotates. The alignment pins 402 are formed on the bottom plate 420 and configured to mate with openings 426 in the top plate 404 to ensure that all of the recesses 422 and the openings 408 are in alignment with the drive wheels 414 sandwiched therebetween. In addition, the use of multiple drive wheels 414 allows the driving force for the catheter 204 to be distributed across multiple drive wheels 414. The internal diameter of the catheter 204 is not reduced or crushed by the drive wheels 414, because less compressive force is needed to drive the catheter 204 as the frictional forces acting on the catheter 204 by the multiple drive wheels 414 is increased.

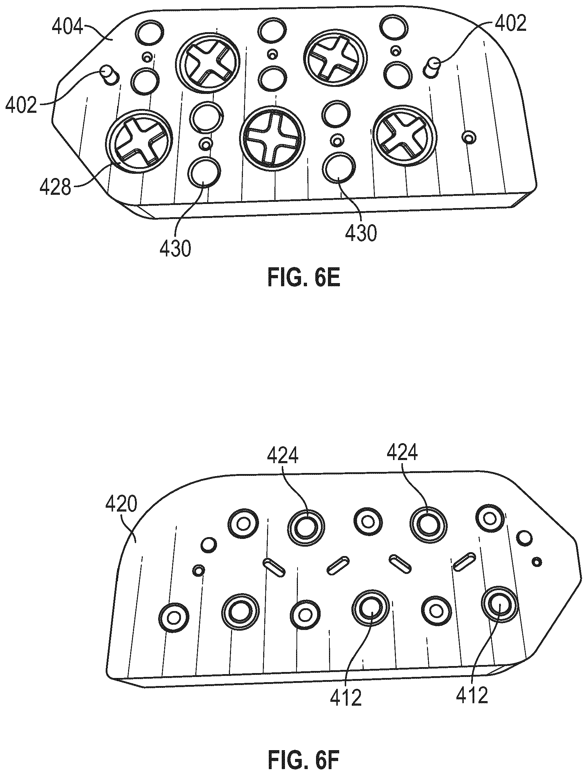

FIG. 6D depicts bearings 428, such as ball or roller bearings, in the opening 408. However, sliding bearings made of, for example, PTFE or Delrin may also be used in place of the bearings 428 and 424. FIGS. 6E and 6F depict the top plate 404 and the bottom plate 420, respectively with the sliding bearings employed rather than ball or roller bearings for bearings 428 and 424. FIG. 6E also shows attachment devices 430 and the alignment pins 402 extending though the top plate 404. As will be described in greater detail below, this allows the Z-drive quick connect to be aligned with and secure to a Z-drive 600.

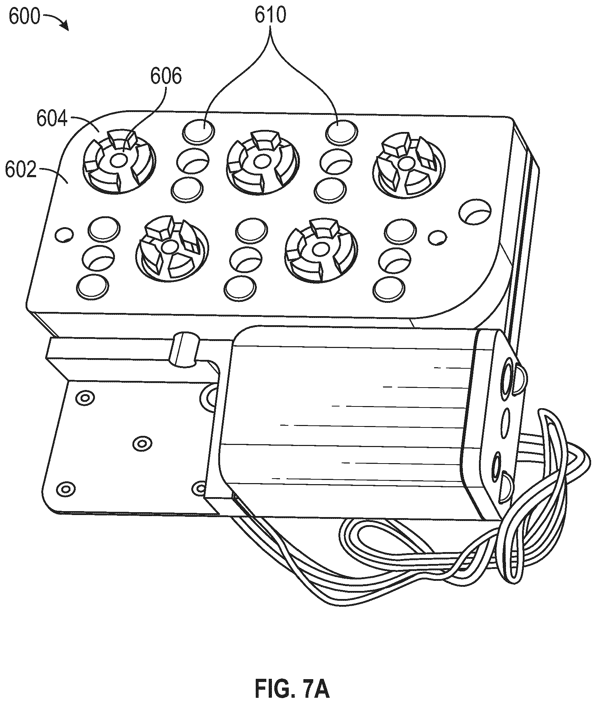

FIG. 7A depicts one aspect of a Z-drive 600 in accordance with the disclosure. The Z-drive 600 includes a top plate 602 with openings 604, through which drivers 606 (similar to drivers 508) extend. The drivers 606 are configured to mate with the rotary interfaces 406 of the Z-drive quick connect 400. The drivers 606 are mounted on shafts (not shown) connected to a gear train (also not shown) to enable their rotation. The alignment pins 402 of the Z-drive quick connect are received in openings 608 in the top plate 602 of the Z-drive. And the attachment devices 430 of the Z-drive quick connect mate with corresponding attachment devices 610 in the top plate. A motor (not shown in FIG. 7A) mechanically interacts with each driver 606, so that all five of the drivers can rotate in a complementary direction. As will be appreciated the drivers 606 on one side will all rotate in an opposite direction from the drivers 606 on the opposite side if the Z-drive 600. Rotation of the drivers 606 causes the rotary interfaces on the Z-drive quick connect to rotate the drive wheels 414. The catheter 204 secured between the drive wheels is then either advanced or retracted into or from the patient, depending on the direction of rotation of the motor, the gear train (not shown) and the interaction of the drivers 606 and the rotary interfaces 606.

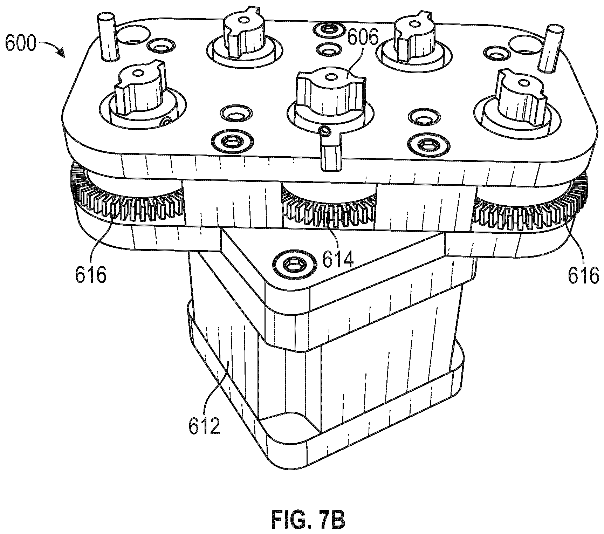

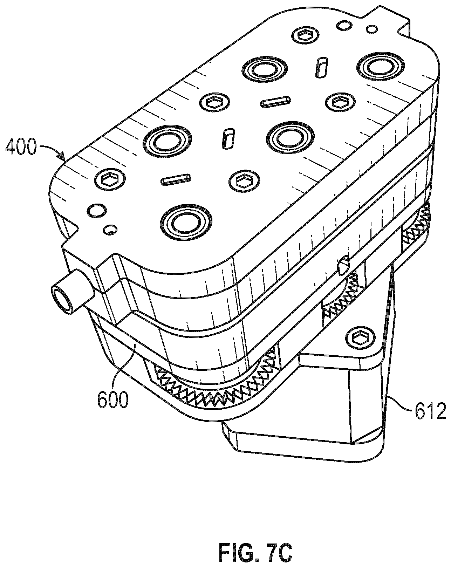

FIG. 7B depicts an alternative configuration of the Z-drive 600. The motor 612 can be seen below the Z-drive can connects via a shaft (not shown) to a drive gear 614. A first driver 606 is mounted on the shaft and is directly driven by the motor 612. The other four drivers 606 are mounted on shafts (not shown), each of which has a driven gear 616 connected thereto. Those of skill in the art will recognize that idler gears (not shown) will be necessary between at least two of the other four driven gears 616 to ensure proper rotation of the drivers 606. FIG. 7C depicts this alternative configuration of Z-drive 600 with a Z-drive quick connect 400.

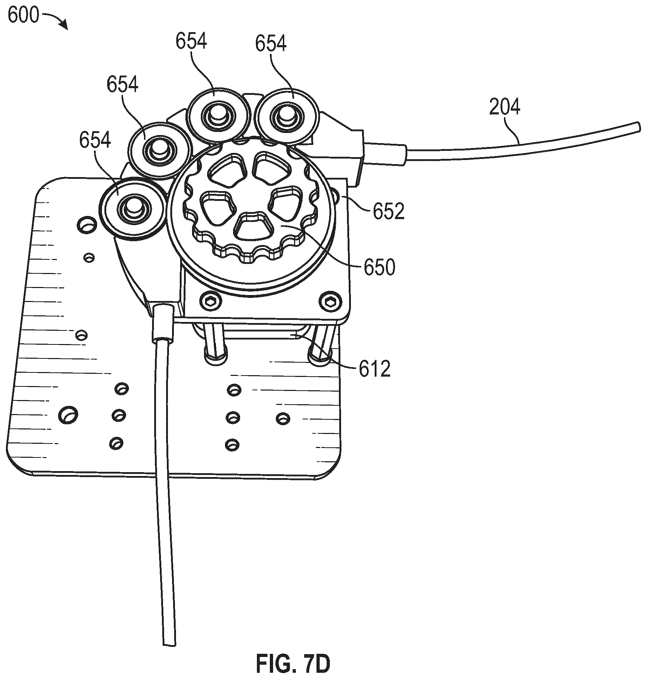

FIG. 7D depicts yet a further alternative Z-drive 600. The Z-drive 600 of FIG. 7D enables Z-direction driving of the catheter 204 without requiring a Z-drive quick connect 400. Instead, the motor 612 is connected to a single drive wheel 650. The single drive wheel 650 includes a frictional mating surface 652 having a generally arcuate shape configured to receive the catheter 204. Opposite the single drive wheel 650 are a plurality of idler wheels 654. Each idler wheel 654 has a frictional mating surface (not shown) with an arcuate shape that substantially conforms to the catheter 204. The two frictional mating surfaces force the catheter 204 to advance or retract the catheter 204 (e.g., drive in the Z-direction) without crushing or altering the internal diameter of the catheter 204.

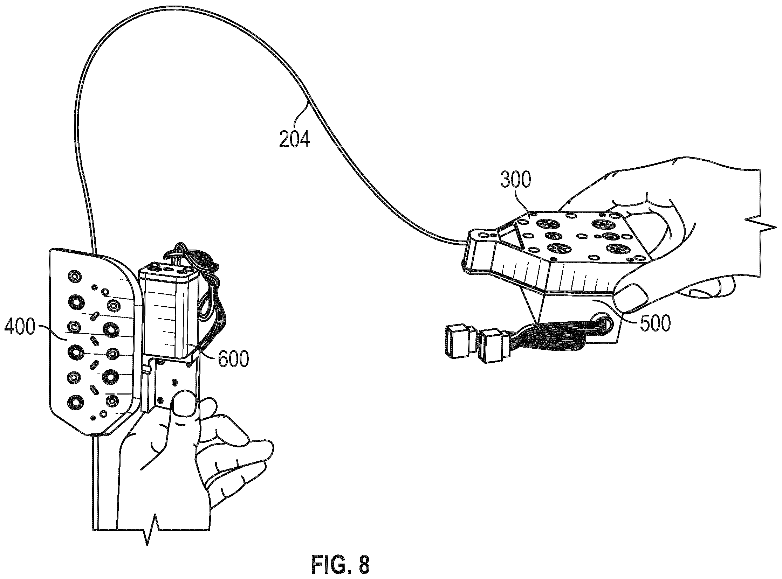

FIG. 8 depicts the entire catheter drive system 200 in accordance with this disclosure. In operation, the Z-drive quick connect 400 can be connected to the Z-drive 600 and the catheter quick connect 300 can be connected to the motor pack 500. In practice, the Z-drive and the Z-drive quick connect will be mounted on a fixed point (e.g, a rigid arm) connected to or proximate a hospital bed on which the patient is located. The catheter quick connect 300 and the motor pack 500 may be mounted on a movable arm (not shown) that provides little or no resistance to motion in the direction along the length of the catheter 204. Alternatively, the catheter quick connect 300 and motor pack 500 can also be mounted on a fixed arm so long as sufficient slack (as shown) is provided to allow Z-direction travel of the catheter 204. As noted above, the Z-drive quick connect 400, catheter 204, and catheter quick connect may be a disposable or a reprocessable/recyclable item. Further, by disconnecting the catheter 204 from the Z-drive and the motor pack 500, changes in size of the catheter 204 do not impact Z-drive 600 and motor pack 500.

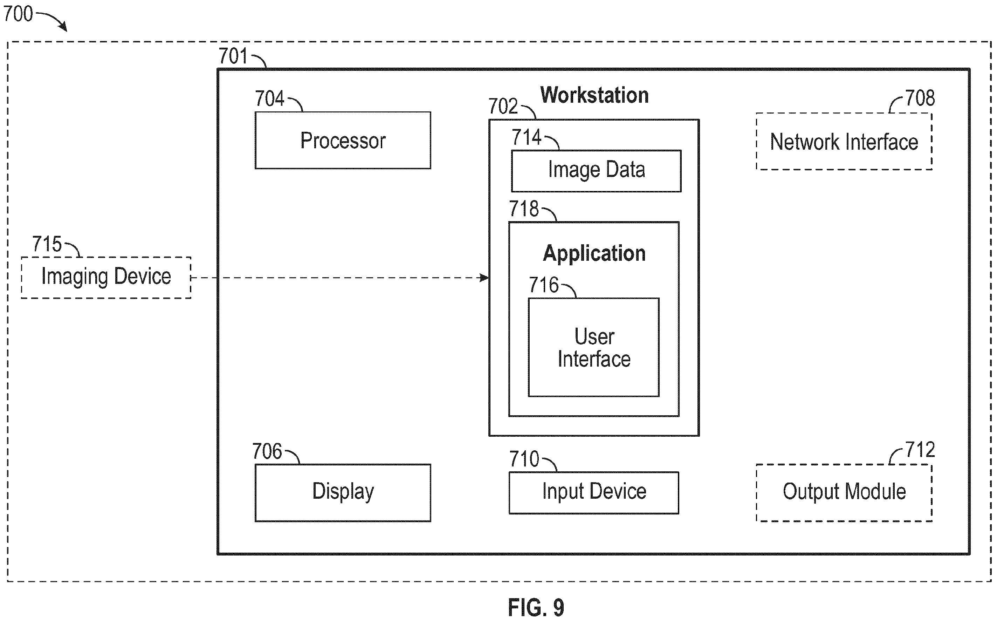

Reference is now made to FIG. 9, which is a schematic diagram of a system 700 configured for use with the devices and systems of the disclosure. System 700 may include a workstation 701, and optionally an imaging device 715 (e.g., a fluoroscope, ultrasound device, or CBCT device). In some embodiments, workstation 701 may be coupled with imaging device 715, directly or indirectly, e.g., by wireless communication. Workstation 701 may include a memory 702, a processor 704, a display 706 and an input device 710. Processor or hardware processor 704 may include one or more hardware processors. Workstation 701 may optionally include an output module 712 and a network interface 708. Memory 702 may store an application 718 and image data 77. Application 718 may include instructions executable by processor.

Application 718 may further include a user interface 716. Image data 714 may include the CT scans, the generated fluoroscopic 3D reconstructions of the target area and/or any other fluoroscopic image data and/or the generated one or more slices of the 3D reconstruction. Processor 704 may be coupled with memory 702, display 706, input device 710, output module 712, network interface 708 and imaging device 715. Workstation 701 may be a stationary computing device, such as a personal computer, or a portable computing device such as a tablet computer. Workstation 701 may embed a plurality of computer devices.

Memory 702 may include any non-transitory computer-readable storage media for storing data and/or software including instructions that are executable by processor 704 and which control the operation of workstation 701 and, in some embodiments, may also control the operation of imaging device 715. Imaging device 715 may be used to capture a sequence of fluoroscopic images based on which the fluoroscopic 3D reconstruction is generated and to capture a live 2D fluoroscopic view according to this disclosure. In an embodiment, memory 702 may include one or more storage devices such as solid-state storage devices, e.g., flash memory chips. Alternatively, or in addition to the one or more solid-state storage devices, memory 702 may include one or more mass storage devices connected to the processor 704 through a mass storage controller (not shown) and a communications bus (not shown).

Although the description of computer-readable media contained herein refers to solid-state storage, it should be appreciated by those skilled in the art that computer-readable storage media can be any available media that can be accessed by the processor 704. That is, computer readable storage media may include non-transitory, volatile, and non-volatile, removable, and non-removable media implemented in any method or technology for storage of information such as computer-readable instructions, data structures, program modules or other data. For example, computer-readable storage media may include RAM, ROM, EPROM, EEPROM, flash memory or other solid-state memory technology, CD-ROM, DVD, Blu-Ray or other optical storage, magnetic cassettes, magnetic tape, magnetic disk storage or other magnetic storage devices, or any other medium which may be used to store the desired information, and which may be accessed by workstation 1001.

Application 718 may, when executed by processor 704, cause display 706 to present user interface 716. User interface 716 may be configured to present to the user a single screen including a three-dimensional (3D) view of a 3D model of a target from the perspective of a tip of a medical device, a live two-dimensional (2D) fluoroscopic view showing the medical device, and a target mark, which corresponds to the 3D model of the target, overlaid on the live 2D fluoroscopic view. User interface 716 may be further configured to display the target mark in different colors depending on whether the medical device tip is aligned with the target in three dimensions. The application may further be configured to receive inputs from a user and enable activation of one or more of the motors of the catheter drive system 200 and articulate or drive the catheter 204 within the patient to a desired location or orientation (e.g., to a point within the patient identified in a navigation plan, as described herein, above).

Network interface 708 may be configured to connect to a network such as a local area network (LAN) consisting of a wired network and/or a wireless network, a wide area network (WAN), a wireless mobile network, a Bluetooth network, and/or the Internet. Network interface 708 may be used to connect between workstation 701 and imaging device 715. Network interface 708 may also be used to receive image data 714. Input device 710 may be any device by which a user may interact with workstation 701, such as, for example, a mouse, keyboard, foot pedal, touch screen, and/or voice interface. Output module 712 may include any connectivity port or bus, such as, for example, parallel ports, serial ports, universal serial busses (USB), or any other similar connectivity port known to those skilled in the art. From the foregoing and with reference to the various figures, those skilled in the art will appreciate that certain modifications can be made to the disclosure without departing from the scope of the disclosure.

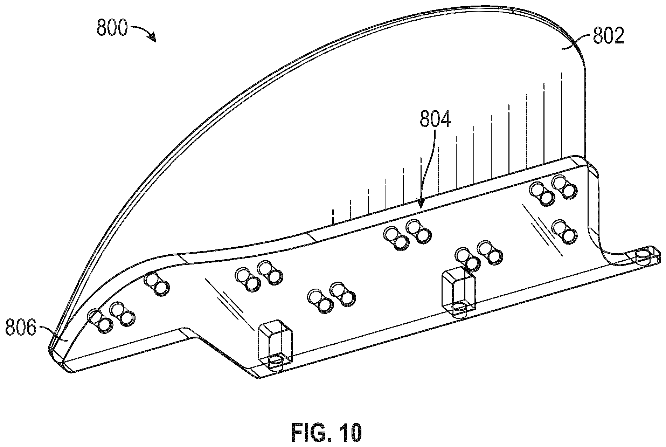

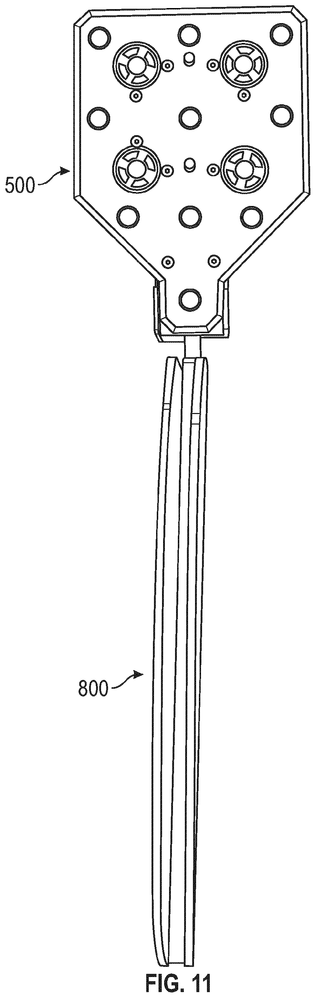

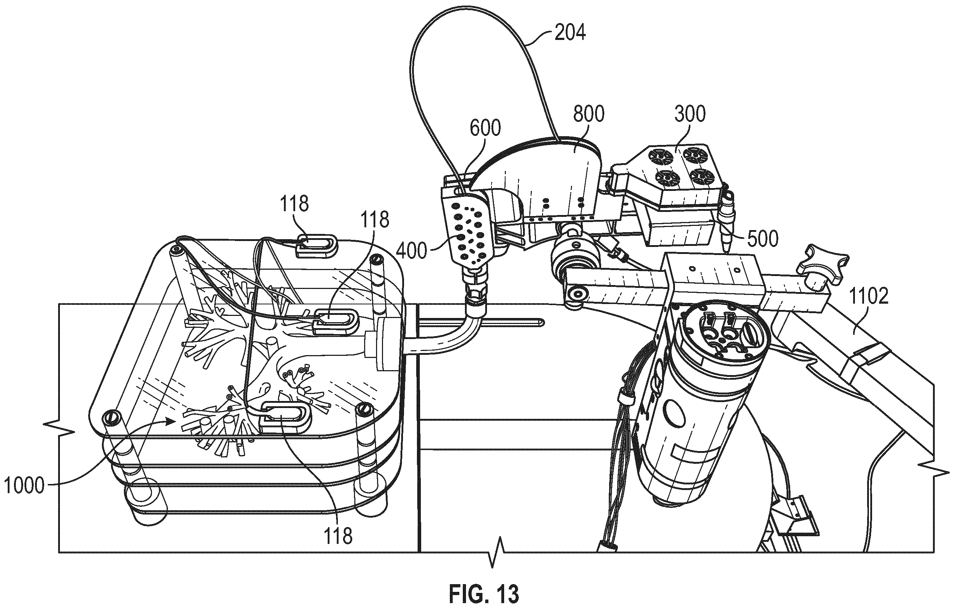

FIG. 10 depicts a partial cut-away view of a slack management guide 800 in accordance with a further aspect of the disclosure. The slack management guide 800 includes a pair of angled guide plates 802 (only one shown in FIG. 10). The angled guide plates 802 are configured to angle away from a profiled base 804 plate and receive the catheter 204 therebetween. The angled guide plates 802 prevent the slack in the catheter 204 from becoming twisted or looping between the Z-drive quick connect 400 and the catheter quick connect 300. The profiled base plate 804 is configured with an arcuate distal end 806, the arcuate distal end 806 prevents kinking of the catheter 204 as the catheter 204 is advanced into the patient. The arcuate distal end 806 ensures that the catheter at maximum extension (Z-direction) into the patient assumes the same shape as the arcuate distal end 806. The top surface 810 of the profiled base plate 804 has a width that is substantially the same dimension as the outer diameter of the catheter 204. This allows, (as shown in FIG. 13) for a proximal portion of the catheter 204 to be wedged between the two angled guide plates 802. The distal portion of the catheter 204 is secured in the Z-drive quick connect, and the slack in the catheter 204 is thus managed between these two points. As the catheter 204 is advanced the slack is reduced until the catheter 204 lies against the top surface 810 of the profiled base, which as noted above has an arcuate shape preventing kinking of the catheter 204. FIG. 11 depicts a top view of the slack management guide 800 connected to a motor pack 500.

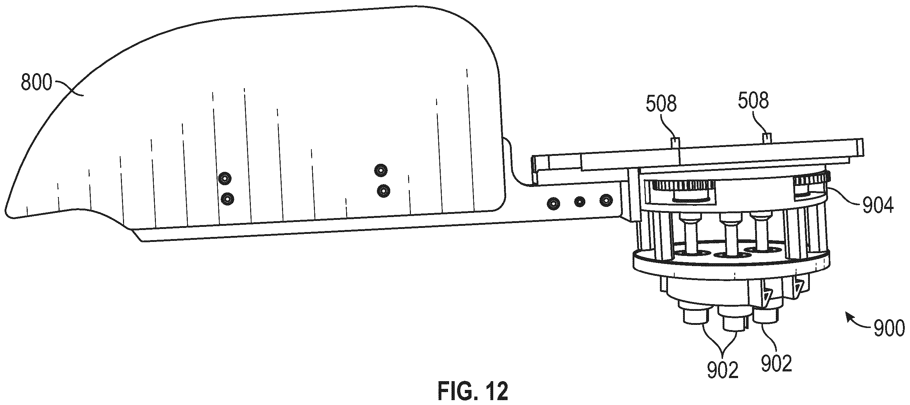

FIG. 12 depicts a side view of a slack management guide 800 connected to an adapter transmission 900. The adapter transmission 900 is configured to receive input (e.g., via connection to one or more motors) on the shafts 902. The shafts 902 are connected via a gear box 904 to the drives 508 which extend through a top surface of the transmission 900 and interact with a catheter quick connect 300, as described above in connection with FIG. 5A.

FIG. 13 depicts an implementation of the disclosure incorporating many of the features described herein above and configured for navigation of the lungs of a patient. FIG. 13 employs a lung model 1000. On the lung model 1000 are the reference sensors 118. The lung model 1000 is placed on the transmitter mat 120. As noted above, the transmitter mat 120 generates magnetic fields that are detected by the reference sensors 118 and a sensor 104/126 in the catheter 204. The disposable catheter system 202, including catheter 204, catheter quick connect 300, and the Z-drive quick connect 400, are driven by motor pack 500 and Z-drive 600. As is shown the motor pack 500 is mounted to a support arm 1002. The support arm 1002 also supports a slack management guide 800 and the Z-drive 600. Both the Z-drive 600 and the motor pack 500 may be connected to a workstation 701, as described, which directs signals to the motors (e.g., 512) to drive the drive wheel 414 of the Z-drive quick connect 400 and the rotary interfaces 334 of the catheter quick connect 300 to advance, retract and articulate the distal portion of the catheter 204. The slack management guide 800 ensures that the catheter 204 is managed and prevented from tangling or becoming kinked as the catheter 204 is driven into the model airways of the model 1000. With the advancement of the catheter 204 into the model 1000 the magnetic fields generated by the transmitter mat 118 are detected by sensor 104/126 on the distal portion of the catheter 204 and the location of the distal portion of the catheter 204 within the model airways can be displayed in a 3D model or images on a display 706 (e.g., in a user interface 716).

The disposable catheter system 202 in connection with the motorized drive system (Z-drive 600 and motor pack 500) enables the essentially hands-free motorized advancement and articulation of the catheter 204. One challenge of current hand-held systems is that to hold an endoscope 108 and advance a catheter 102 and then hold the catheter 102 steady once a location within the airways of the patient while manipulating another tool such as a biopsy or therapy tool which is to traverse the inner lumen of the catheter 204. In contrast, with the disposable catheter system 202 mounted on the rigid support arm 1002, only biopsy or therapy tool needs to be manipulated by the clinician and the distal portion of the catheter 204 is maintained in place in the patient.

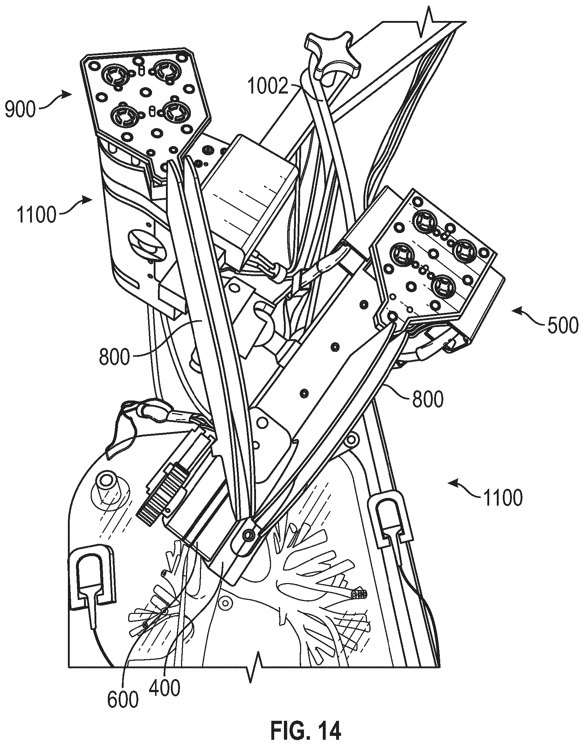

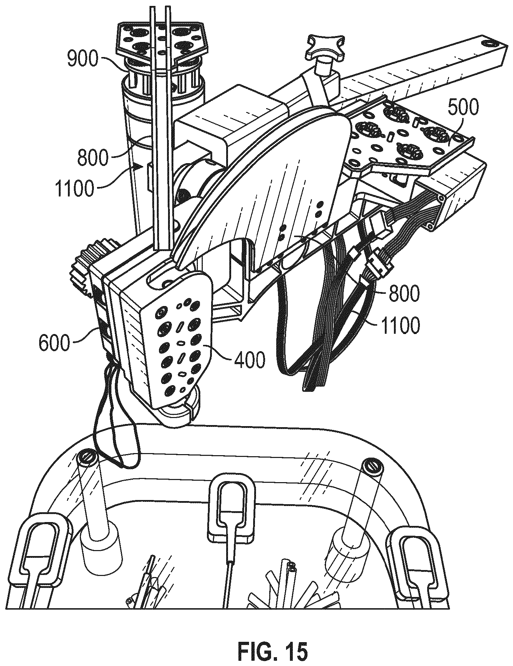

FIG. 14 depicts yet a further aspect of the disclosure depicting a rigid arm 1002 configured to mount two catheter drive systems 1100. A first catheter drive system 1100 includes a motor pack 500, a slack management guide 800, and a Z-drive quick connect 400 mounted to a Z-drive 600. A second catheter drive system 1100 includes an alternative motor pack 1102 configured to mate with a transmission 900 (FIG. 12) including a second slack management guide 800. In this embodiment, a single Z-drive quick connect 400 is employed. In accordance with the disclosure, a first catheter 204 is navigated using one of the first or second drive systems 1100, for example a catheter 204 to be employed for a biopsy procedure. With the retrieval of the first catheter 204, a second catheter 204 may be inserted into the Z-drive quick connect 400, for example a catheter 204 configured for application of a therapy and navigated to a desired location in the patient. The use of multiple catheter drive systems 1100 can streamline aspects of the procedure. FIG. 15 depicts an alternative view of the rigid arm 1002 and two catheter drive systems 1100.

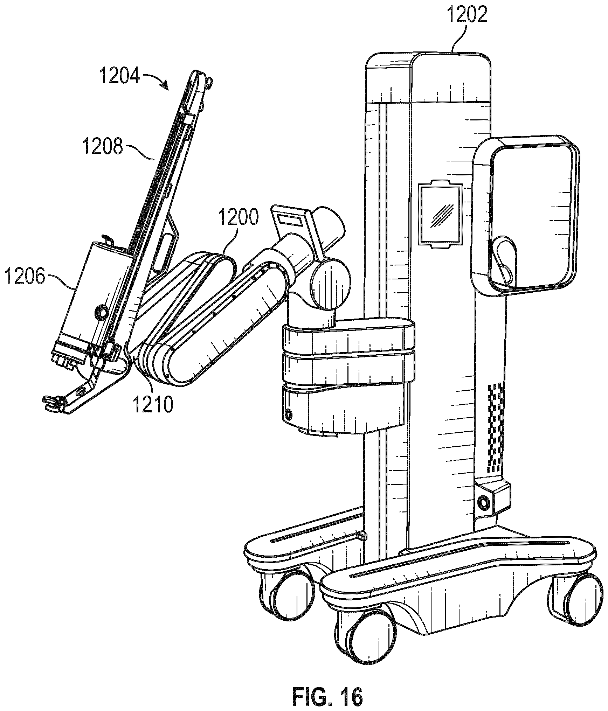

FIG. 16 depicts a surgical robotic arm 1200 mounted on a mobile cart 1202. Mounted on the robotic arm 1200 is a holder 1204 configured to receive and support an instrument drive unit (IDU) 1206. As described in greater detail below the IDU 1206 is configured to couple to a robotic catheter drive system 1300. The holder 1204 includes a sliding mechanism 1208, which is configured to move the IDU 1206 along the second longitudinal axis defined by the holder 1204. The holder 1204 also includes a joint 1210, which allows holder 1204 to rotate to one or more links of the surgical robotic arm.

The robotic catheter drive system 1300 is depicted in FIG, 17. On a distal end of the robotic catheter drive system 1300 is a Z-drive 600, and as shown a Z-drive quick connect 400 is mounted to the Z-drive 600. A rail 1302 connects the Z-drive 600 to a motor adapter 1304. The motor adapter 1304 is configured to mate with the IDU 1206 and transfer motor motion from the IDU 1206 to a robotic catheter quick connect 1306. The robotic catheter quick connect 1306 includes a plurality of pull wires and is configured to mate with the motor adapter 1304 so that motorized motion can be transferred from the IDU 1206 though the motor adapter 1304 to the robotic catheter quick connect 1306 (in a similar fashion to that described above with respect to the motor pack 500 and catheter quick connect 300) to enable articulation of the catheter 204 extending from the robotic catheter quick connect 1306. Also mounted to the rail 1302 is a slack management guide 800 which is configured to receive the catheter 204 and prevent both twisting and kinking of the catheter 204. A shaft (not shown) extends through the rail 1302 to transfer motor motion from the IDU 1206 to the Z-drive 600 and the Z-drive quick connect 400 to advance the catheter 204 into and retract the catheter 204 from the patient.

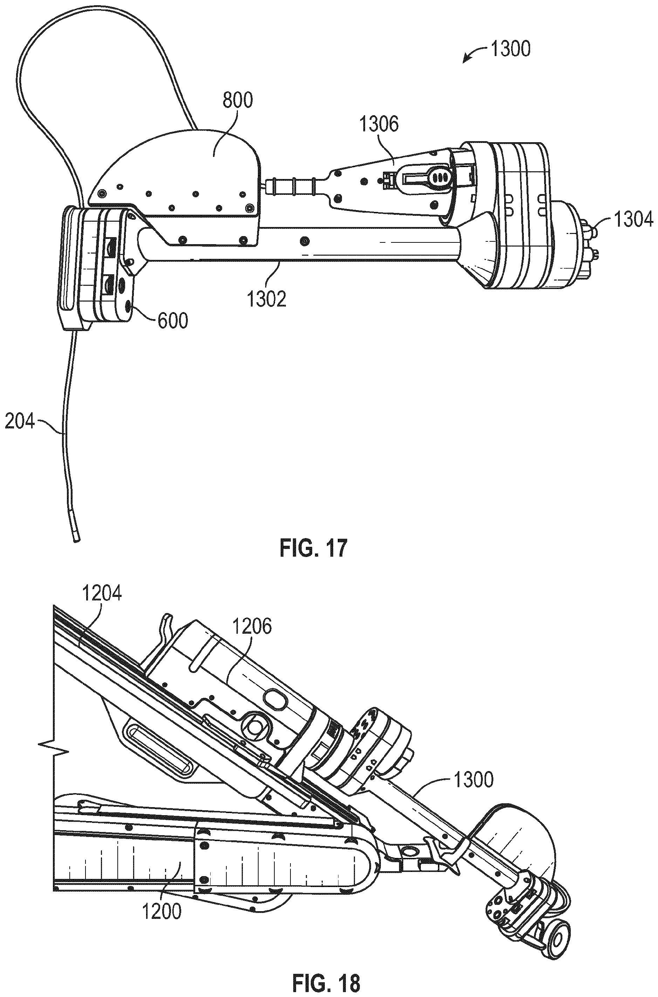

FIG. 18 depicts the robotic catheter drive system 1300 mounted on and IDU 1206 that is slidingly secured on the holder 1204. The holder 1204 is rotatably mounted to a link of the robotic arm 1200. In practice, the robotic arm 1200 and the holder 1204 can be manipulated to reach a desired location relative to the patient. In the case of lung navigation this desired position is an orientation where the z-drive 600 and the z-drive quick connect 400 are oriented such that the catheter 204 is in line with the patient's mouth and oriented to drive the catheter 204 in the direction of the patient's trachea. Once the robotic arm 1200 is so positioned, the IDU 1206 receives signals from a navigation application (e.g., on workstation 701) to advance and articulate the catheter through the airways of the patient. The patient may be positioned on a transmitter mat 114 and sensors 104/126 on the catheter 204 may detect the magnetic fields generated by the transmitter mat. The navigation application determines a location of the sensor 104/126 within the patient and presents one or more user interfaces depicting the catheter 204 within the patient with reference to a 3D model or other images of the patient. Where a navigation plan has been developed, the catheter 204 may be navigated, following the navigation plan, to a desired location within the patient for biopsy or therapy. The progress of the navigation may be continually updated in the user interface until the catheter 204 arrives at the desired location.

### EXAMPLES

The features and aspects of the disclosure are further described in connection with the following examples.
Example - 1 - A catheter drive system comprising:
   a motor pack including a plurality of motors, each motor connected to a driver;
   a catheter quick connect, including a catheter mounted therein, and a plurality of rotary interfaces configured to mate with the drivers;
   a plurality of spindles, each spindle coupled to one of the rotary interfaces such that rotation of the rotary interface is transferred to the spindle;
   a plurality of pull-wires, each pull-wire connected to one of the spindles and extending into a distal portion of the catheter, wherein rotation of the spindle in a first rotational direction retracts the pull-wire onto the spindle articulating the catheter in a first direction, and rotation of the spindle in a second rotational direction relaxes the catheter allowing another pull-wire to articulate the catheter in second direction opposite the first direction.
   a Z-drive quick connect configured to receive the catheter and move the catheter along its longitudinal axis;
   a Z-drive including at least one motor and a plurality of drivers mechanically coupled to the motor, each driver coupleable to a corresponding rotary interface on the Z-drive quick connect, wherein rotation of the drivers by the motor is transferred to the rotary interfaces of the Z-drive quick connect.
Example 2 - The catheter drive system of example 1, wherein the catheter quick connect and the motor pack each include a plurality of attachment devices configured to affix the catheter quick connect to the motor pack.
Example 3 - The catheter drive system of any of the preceding examples, wherein the motor pack includes an alignment device configured to align the motor pack and the catheter quick connect. Example 4 - The catheter drive system of example 3, wherein the alignment device is a post configured to mate with an opening on the catheter quick connect.
Example 5 - The catheter drive system of any of the preceding examples, wherein the Z-drive quick connect and the Z-drive each include a plurality of attachment devices configured to affix the catheter quick connect to the motor pack.
Example 6 - The catheter drive system of claim of any of the preceding examples, wherein the Z-drive quick connect includes an alignment device configured to align the motor pack and the catheter quick connect.
Example 7 - The catheter drive system of example 6, wherein the alignment device is a post configured to mate with an opening on the Z-drive.
Example 8 - The catheter drive system of any of the preceding examples, wherein the Z-drive directly connects to a drive gear connected to a first of the plurality of drivers, and the remainder of the plurality of drivers are operably connected to the drive gear by driven gears.
Example 9 - The catheter drive system of any of the preceding examples, wherein the Z-drive quick connect includes a plurality of drive wheels, wherein the drive wheels are configured to frictionally drive the catheter when the drive wheels rotate.
Example 10 - The catheter drive system of any of the preceding examples, wherein the catheter quick connect is a four pull-wire device.
Example 11 - The catheter drive system of any of the preceding examples, further including a slack management guide.
Example 12 - The catheter drive system of example 11, wherein the slack management guide includes a pair of angled guide plates and a profiled base plate.
Example 13 - The catheter drive system of example 12, wherein the profiled base plate includes a distal end configured to prevent kinking of the catheter.
Example 14 - The catheter drive system of example 12, wherein the angled guide plates are configured to secure a portion of the catheter therebetween to prevent twisting of the catheter.
Example 15 - A robotic catheter drive system including a Z-drive mounted at a distal end of a rail and configured to receive a Z-drive quick connect, a motor adapter mounted on a proximal end of the rail, the motor adapter configured to mate with an instrument drive unit (IDU) and transmit motorized motions from the IDU to a robotic catheter quick connect and the Z-drive, and a slack management guide mounted on the rail between the motor adapter and the Z-drive and configured to manage slack in a catheter.
Example 16 - The robotic catheter drive system of example 15, further including a disposable catheter system, the disposable catheter system including a Z-drive quick connect connected to a distal portion of a catheter and the Z-drive and a robotic catheter quick connect on a proximal portion of the catheter and the motor adapter.
Example 17 - The robotic catheter drive system of example 15, wherein the slack management guide includes a pair of angled guide plates and a profiled base plate.
Example 18 - The robotic catheter drive system of example 16, wherein the profiled base plate includes a distal end configured to prevent kinking of the catheter.
Example 19 - The robotic catheter drive system of example 16, wherein the pair of angled guide plates are configured to secure at least a portion of the catheter between them to prevent twisting of the catheter.
Example 20 - The robotic catheter drive system of example 15, further comprising a shaft mechanically coupling the adapter and the Z-drive.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments.

The invention may be described by reference to the following numbered paragraphs:-
1. A catheter drive system comprising:
   a motor pack including a plurality of motors, each motor connected to a driver;
   a catheter quick connect, including a catheter mounted therein, and a plurality of rotary interfaces configured to mate with the drivers;
   a plurality of spindles, each spindle coupled to one of the rotary interfaces such that rotation of the rotary interface is transferred to the spindle;
   a plurality of pull-wires, each pull-wire connected to one of the spindles and extending into a distal portion of the catheter, wherein rotation of the spindle in a first rotational direction retracts the pull-wire onto the spindle articulating the catheter in a first direction, and rotation of the spindle in a second rotational direction relaxes the catheter allowing another pull-wire to articulate the catheter in second direction opposite the first direction.
   a Z-drive quick connect configured to receive the catheter and move the catheter along its longitudinal axis;
   a Z-drive including at least one motor and a plurality of drivers mechanically coupled to the motor, each driver coupleable to a corresponding rotary interface on the Z-drive quick connect, wherein rotation of the drivers by the motor is transferred to the rotary interfaces of the Z-drive quick connect.
2. The catheter drive system of paragraph 1, wherein the catheter quick connect and the motor pack each include a plurality of attachment devices configured to affix the catheter quick connect to the motor pack.
3. The catheter drive system of paragraph 1, wherein the motor pack includes an alignment device configured to align the motor pack and the catheter quick connect.
4. The catheter drive system of paragraph 3, wherein the alignment device is a post configured to mate with an opening on the catheter quick connect.
5. The catheter drive system of paragraph 1, wherein the Z-drive quick connect and the Z-drive each include a plurality of attachment devices configured to affix the catheter quick connect to the motor pack.
6. The catheter drive system of paragraph 1, wherein the Z-drive quick connect includes an alignment device configured to align the motor pack and the catheter quick connect.
7. The catheter drive system of paragraph 3, wherein the alignment device is a post configured to mate with an opening on the Z-drive.
8. The catheter drive system of paragraph 1, wherein the Z-drive directly connects to a drive gear connected to a first of the plurality of drivers, and a remainder of the plurality of drivers are operably connected to the drive gear by driven gears.
9. The catheter drive system of paragraph 1, wherein the Z-drive quick connect includes a plurality of drive wheels, wherein the drive wheels are configured to frictionally drive the catheter when the drive wheels rotate.
10. The catheter drive system of paragraph 1, wherein the catheter quick connect is a four pull-wire device.
11. The catheter drive system of paragraph 1, further comprising a slack management guide.
12. The catheter drive system of paragraph 11, wherein the slack management guide includes a pair of angled guide plates and a profiled base plate.
13. The catheter drive system of paragraph 12, wherein the profiled base plate includes a distal end configured to prevent kinking of the catheter.
14. The catheter drive system of paragraph 12, wherein the angled guide plates are configured to secure a portion of the catheter therebetween to prevent twisting of the catheter.
15. A robotic catheter drive system comprising:
   a Z-drive mounted at a distal end of a rail and configured to receive a Z-drive quick connect;
   a motor adapter mounted on a proximal end of the rail, the motor adapter configured to mate with an instrument drive unit (IDU) and transmit motorized motions from the IDU to a robotic catheter quick connect and the Z-drive; and
   a slack management guide mounted on the rail between the motor adapter and the Z-drive and configured to manage slack in a catheter.
16. The robotic catheter drive system of paragraph 15, further comprising a disposable catheter system, the disposable catheter system including a Z-drive quick connect connected to a distal portion of a catheter and the Z-drive and a robotic catheter quick connect on a proximal portion of the catheter and the motor adapter.
17. The robotic catheter drive system of paragraph 15, wherein the slack management guide includes a pair of angled guide plates and a profiled base plate.
18. The robotic catheter drive system of paragraph 16, wherein the profiled base plate includes a distal end configured to prevent kinking of the catheter.
19. The robotic catheter drive system of paragraph 16, wherein the pair of angled guide plates are configured to secure at least a portion of the catheter between them to prevent twisting of the catheter.
20. The robotic catheter drive system of paragraph 15, further comprising a shaft extending through the rail and mechanically coupling the motor adapter and the Z-drive.

## Claims

1. A catheter drive system comprising:
a motor pack including a plurality of motors, each motor connected to a driver;
a catheter quick connect, including a catheter mounted therein, and a plurality of rotary interfaces configured to mate with the drivers;
a plurality of spindles, each spindle coupled to one of the rotary interfaces such that rotation of the rotary interface is transferred to the spindle;
a plurality of pull-wires, each pull-wire connected to one of the spindles and extending into a distal portion of the catheter, wherein rotation of the spindle in a first rotational direction retracts the pull-wire onto the spindle articulating the catheter in a first direction, and rotation of the spindle in a second rotational direction relaxes the catheter allowing another pull-wire to articulate the catheter in second direction opposite the first direction.
a Z-drive quick connect configured to receive the catheter and move the catheter along its longitudinal axis;
a Z-drive including at least one motor and a plurality of drivers mechanically coupled to the motor, each driver coupleable to a corresponding rotary interface on the Z-drive quick connect, wherein rotation of the drivers by the motor is transferred to the rotary interfaces of the Z-drive quick connect.

2. The catheter drive system of claim 1, wherein the catheter quick connect and the motor pack each include a plurality of attachment devices configured to affix the catheter quick connect to the motor pack.

3. The catheter drive system of any of claims 1-2, wherein the motor pack includes an alignment device configured to align the motor pack and the catheter quick connect.

4. The catheter drive system of claim 3, wherein the alignment device is a post configured to mate with an opening on the catheter quick connect.

5. The catheter drive system of any of claims 1-4, wherein the Z-drive quick connect and the Z-drive each include a plurality of attachment devices configured to affix the catheter quick connect to the motor pack.

6. The catheter drive system of any of claims 1-5, wherein the Z-drive quick connect includes an alignment device configured to align the motor pack and the catheter quick connect.

7. The catheter drive system of claim 3, wherein the alignment device is a post configured to mate with an opening on the Z-drive.

8. The catheter drive system of any of claims 1-7, wherein the Z-drive directly connects to a drive gear connected to a first of the plurality of drivers, and a remainder of the plurality of drivers are operably connected to the drive gear by driven gears.

9. The catheter drive system of any of claims 1-8, wherein the Z-drive quick connect includes a plurality of drive wheels, wherein the drive wheels are configured to frictionally drive the catheter when the drive wheels rotate.

10. The catheter drive system of any of claims 1-9, wherein the catheter quick connect is a four pull-wire device.

11. The catheter drive system of any of claims 1-10, further comprising a slack management guide.

12. The catheter drive system of any of claim 11, wherein the slack management guide includes a pair of angled guide plates and a profiled base plate.

13. The catheter drive system of claim 12, wherein the profiled base plate includes a distal end configured to prevent kinking of the catheter.

14. The catheter drive system of claim 12, wherein the angled guide plates are configured to secure a portion of the catheter therebetween to prevent twisting of the catheter.

15. The catheter drive system of any of claims 1-14, mounted on a surgical robot.
